# EUROPÄISCHE PATENTANMELDUNG

(11) **EP 4 353 313 A2**
(43) Veröffentlichungstag der Anmeldung: **17.04.2024**
(21) Anmeldenummer: 23197283.7
(22) Anmeldetag: 21.08.2022
(51) Int. Cl.: A61P 13/12

(54) **KRÄUTERMISCHUNG ZUR VERBESSERTEN NIEREN-UND BLASENFUNKTION UND GEGEN BLASENSCHWÄCHE**

(62) Teilanmeldung aus: 22191354.4
(71) Anmelder: Sun, Xiaochun, 12347 Berlin (DE)
(72) Erfinder: Sun, Xiaochun, 12347 Berlin (DE)

(57) **Zusammenfassung**

Die Erfindung betrifft mehrere Kräutermischungen basierend auf TCM (Traditioneller Chinesischer Medizin), zur Verstärkung der Nieren- und Blasenfunktion gegen Blasenschwäche, sowie zur Leistungssteigerung und Blutverdünnung. Die Erfindung unterstützt den Schliess- und Ringmuskel zu stärken für die verbesserte Kontrolle beim Harngang.

Die Erfindung umfasst mehrere Mischungen von 5-6 Zutaten. Diese werden zu Granulat verarbeitet oder als Extrakt gewonnen und oral eingenommen.
1. Astragali radix 7-12g, Paeonia lactiflora Pall. 7-15g, Crataegus pinnatifida Bunge 7-12g, Psoralea corylifolia Linn.5-12 g, Rosa laevigata Michx. 5-12g, Cornus officinalis Sieb. et Zucc. 5-12g
Weitere Rezepte
2. Piperis Longi Fructus 7-12g, Persicae semen 7-15g, Astragali radix, 5-9g, Psoraleae fructus 5-9g, Corni Fructus 7-12g
3. Chuanxiong rhizoma 7-12g, Paeonia lactiflora Pall. 7-15g, Angelicae sinensis radix 5-9g, Cuscutae semen 7-12g, Corni Fructus 5-9g
4. Cinnamomi ramuli 7-12g, Salviae miltiorrhizae radix et rhizoma 7-15g, Angelicae sinensis radix 5-9g, Cuscutae semen 7-12g, Morindae officinalis radix 5-9g
5. Citri reticulatae pericarpium viride 7-12g, Crocus sativus L 3-7g, Angelicae sinensis radix 5-9g, Rosae laevigatae Fructus 7-12g, Corni Fructus 5-9g
6. Citrus reticulata Blanco7-12g, Rosae rugosae flos 7-12g, Angelicae sinensis radix 5-9g, Alpiniae oxyphyllae fructus 7-12g, Juglans regia 5-9g
7. Pricklyash Peel 7-12g, Mori folium 7-15g, Angelicae sinensis radix 5-9g, Rosa davurica Pall. Fructus 7-12g, Alpiniae oxyphyllae fructus 5-9g Diese Erfindung bezieht sich auf die Zusammensetzung und Extrakt des gleichen.

Wirkung und Fallbeispiele

Es folgen Beispiele von Behandlungen.

Die Kräuterkombination wird zu Tee gemacht. Die Kräuter mit einem halben Liter Wasser 30 Minuten kochen lassen bis auf 250ml und den Aufguss abfüllen. Erneut die Kräuter mit einem halben Liter Wasser 30 Minuten kochen lassen, beide Aufgüsse zusammenmischen. Die Einnahme erfolgt im ersten Monat jeden Tag zwei Mal. Mit einer verlängerten Therapie ab der sechsten Woche, jeden Tag ein oder zwei Mal.

Die Zutaten als Granulat 10 Gramm mit 200ml Wasser aufkochen und 10 Minuten ziehen lassen Die Einnahme erfolgt im ersten Monat jeden Tag zwei Mal. Mit einer verlängerten Therapie ab der sechsten Woche, jeden Tag ein oder zwei Mal.
Rezept 1: Astragali radix 9g, Paeonia lactiflora Pall. 13g, Crataegus pinnatifida Bunge 9g, Psoralea corylifolia Linn. 8g, Rosa laevigata Michx. 5g, Cornus officinalis Sieb. et Zucc. 5g
Rezept 2: Piperis Longi Fructus (9g), Persicae semen (13g), Astragali radix, (9g) , Psoraleae fructus (5g), Corni Fructus (8g)
Rezept 3: Chuanxiong rhizoma (9g), Paeonia lactiflora Pall. (13g), Angelicae sinensis radix (9g), Cuscutae semen (8g), Corni Fructus (5g)
Rezept 4: Cinnamomi ramuli (9g), Salviae miltiorrhizae radix et rhizoma (13g), Angelicae sinensis radix (9g), Cuscutae semen (8g), Morindae officinalis radix (5g)
Rezept 5: Citri reticulatae pericarpium viride (9g), Crocus sativus L (5g), Angelicae sinensis radix (9g), Rosae laevigatae Fructus (8g), Corni Fructus (5g)
Rezept 6: Citrus reticulata Blanco (9g), Rosae rugosae flos (13g), Angelicae sinensis radix (9g) , Alpiniae oxyphyllae fructus (8g) , Juglans regia (5g)
Rezept 7: Pricklyash Peel (9g), Mori folium (13g), Angelicae sinensis radix (9g), Rosa davurica Pall. Fructus (8g), Alpiniae oxyphyllae fructus (5g)
Patient A, weiblich, 47, leidet an Blasenschwäche. Symptome: von halbstündlichem Wasserlassen nach Flüssigkeitseinnahme. Kann den Urin nicht halten und starke Dringlichkeit zum Urinieren.

Nach Einnahme der Kräuter mit Rezept 1 über 3 Tage, deutlich verbessert. Eine Therapie von einem Monat, kann Urin normal regulieren, über eine Therapiedauer von 3 Monaten symptomlos: Kann Urin normal regulieren und uriniert kräftig, fühlt sich körperlich gekräftigt und besitzt mehr Energie für den Alltag.

Patient B, weiblich, 46, leidet an Blasenschwäche. Symptome: Kann den Urin nicht halten und starke Dringlichkeit zum Urinieren, Wasserlassen kurz und kraftlos. Bei Husten Tröpfchenverlust.

Nach Einnahme der Kräuter mit Rezept 1 über 3 Tage, spürt starke Verbesserung. Eine Therapie von einem Monat, kann Urin normal regulieren, über eine Therapiedauer von 3 Monaten symptomlos: Kann Urin normal regulieren und uriniert kräftig, fühlt sich körperlich gekräftigt und besitzt mehr Energie für den Alltag.

Patient C, männlich, 67, leidet an Blasenschwäche mit Prostatavergrösserung. Symptom: Häufiges, kraftloses, kurzes Urinieren, wenig Urin, auch in der Nacht. Nach Einnahme der Kräuter als Granulat mit Rezept 2 über 3 Tage, spürt Verbesserung. Eine Therapie von einem Monat, kann Urin normal regulieren, über eine Therapiedauer von 3 Monaten symptomlos, Eine Therapiedauer von einem halben Jahr, deutliche Verbesserung der Symptome: Uriniert kräftig und viel, reduziertes Erwachen in der Nacht.

Patient D, weiblich, 50, leidet an Blasenschwäche. Symptome: Kann den Urin nicht halten und starke Dringlichkeit zum Urinieren, Wasserlassen kurz, kraftlos und wenig. Bei Lachen Tröpfchenverlust.

Nach Einnahme der Kräuter mit Rezept 2 über 3 Tage, spürt Verbesserung. Eine Therapie von einem Monat, kann Urin normal regulieren, über eine Therapiedauer von 3 Monaten symptomlos, über eine Therapiedauer von einem halben Jahr, danach symptomlos: Kann Urin normal regulieren und uriniert kräftig, fühlt sich körperlich gekräftigt und besitzt mehr Energie für den Alltag.

Patient E, weiblich, 50, leidet an Blasenschwäche. Symptome: Kann den Urin nicht halten und starke Dringlichkeit zum Urinieren, Wasserlassen kurz und wenig, kraftlos.

Nach Einnahme der Kräuter als Granulat mit Rezept 3 über 3 Tage, spürt Verbesserung. Eine Therapie von einem Monat, kann Urin normal regulieren, über eine Therapiedauer von 3 Monaten symptomlos, über eine verlängerte Therapiedauer von einem halben Jahr, fühlt sich körperlich gekräftigt und verjüngt und besitzt mehr Energie für den Alltag.

Patient F, weiblich, 50, leidet an Blasenschwäche. Symptome: Kann den Urin nicht halten und starke Dringlichkeit zum Urinieren, Wasserlassen kurz, kraftlos und kleine Menge. Bei Husten Tröpfchenverlust.

Nach Einnahme der Kräuter als Granulat mit Rezept 3 über 3 Tage, spürt Verbesserung. Eine Therapie von einem Monat, kann Urin normal regulieren, über eine Therapiedauer von 3 Monaten symptomlos, über eine verlängerte Therapiedauer von einem halben Jahr, fühlt sich körperlich gekräftigt und verjüngt und besitzt mehr Energie für den Alltag.

Patient G, weiblich, 51, leidet an Blasenschwäche. Symptome: Kann den Urin nicht halten und starke Dringlichkeit zum Urinieren, Wasserlassen jeweils kurz und wenig, häufige Müdigkeit und Schläfrigkeit.

Nach Einnahme der Kräuter mit Rezept 4 über 3 Tage, spürt Verbesserung. Eine Therapie von einem Monat, kann Urin normal regulieren, über eine Therapiedauer von 3 Monaten symptomlos, über eine verlängerte Therapiedauer von einem halben Jahr, fühlt sich körperlich gekräftigt und verjüngt und besitzt mehr Energie für den Alltag.

Patient H, weiblich, 89, leidet an Blasenschwäche. Symptome: Kann den Urin nicht halten und starke Dringlichkeit zum Urinieren, Wasserlassen jeweils kurz und wenig, Bei Lachen Tröpfchenverlust.

Nach Einnahme der Kräuter als Granulat mit Rezept 4 über 3 Tage, spürt Verbesserung. Eine Therapie von einem Monat, kann Urin normal regulieren, über eine Therapiedauer von 3 Monaten symptomlos, über eine verlängerte Therapiedauer von einem halben Jahr, fühlt sich körperlich gekräftigt und besitzt mehr Energie für den Alltag.

Patient I, weiblich, 66, leidet an Blasenschwäche. Durch Reise und Schlafmangel verursachte Blasenschwäche. Keine Schmerzen und kein Brennen an der Harnröhrenöffnung. Symptome: Dringlichkeit zum Urinieren, Wasserlassen kraftlos, kurz und kleine Menge.

Nach Einnahme der Kräuter mit Rezept 5 über 3 Tage, spürt Verbesserung. Eine Therapie von zwei Wochen kann Urin normal regulieren, verlängerte Therapiedauer von einem Monat, fühlt sich körperlich gekräftigt und verjüngt und besitzt mehr Energie für den Alltag.

Patient J, männlich, 64, leidet an Blasenschwäche. Durch Reise und Schlafmangel verursachte Blasenschwäche. Keine Schmerzen und kein Brennen an der Harnröhrenöffnung. Symptome: Dringlichkeit zum Urinieren, Wasserlassen kraftlos, kurz und kleine Menge.

Nach Einnahme der Kräuter mit Rezept 5 über 3 Tage, spürt Verbesserung. Eine Therapie von zwei Wochen kann Urin normal regulieren, verlängerte Therapiedauer von einem Monat, fühlt sich körperlich gekräftigt und verjüngt und besitzt mehr Energie für den Alltag.

Patient K, weiblich, 91, leidet an Blasenschwäche. Symptome: Kann den Urin nicht halten und starke Dringlichkeit zum Urinieren, Wasserlassen jeweils kurz und wenig, Bei Lachen Tröpfchenverlust.

Nach Einnahme der Kräuter mit Rezept 6 über 3 Tage, spürt Verbesserung. Eine Therapie von einem Monat, kann Urin normal regulieren, über eine Therapiedauer von 3 Monaten symptomlos, über eine verlängerte Therapiedauer von einem halben Jahr, fühlt sich körperlich gekräftigt und besitzt mehr Energie für den Alltag. Patient L, männlich, 29, leidet an Blasenschwäche. Durch Arbeitsstress und Schlafmangel verursachte Blasenschwäche. Keine Schmerzen und kein Brennen an der Harnröhrenöffnung. Symptome: Dringlichkeit zum Urinieren, Wasserlassen kraftlos, kurz und kleine Menge.

Nach Einnahme der Kräuter mit Rezept 6 über 3 Tage, spürt Verbesserung. Eine Therapie von zwei Wochen kann Urin normal regulieren, verlängerte Therapiedauer von einem Monat, fühlt sich körperlich gekräftigt und verjüngt und besitzt mehr Energie für den Alltag.

Patient M, männlich, 55, leidet an Blasenschwäche. Durch Arbeitsstress und Schlafmangel verursachte Blasenschwäche. Keine Schmerzen und kein Brennen an der Harnröhrenöffnung. Symptome: Dringlichkeit zum Urinieren, Wasserlassen kraftlos, kurz und kleine Menge.

Nach Einnahme der Kräuter mit Rezept 7 über 3 Tage, spürt Verbesserung. Eine Therapie von zwei Wochen kann Urin normal regulieren, verlängerte Therapiedauer von einem Monat, fühlt sich körperlich gekräftigt und verjüngt und besitzt mehr Energie für den Alltag.

Patient N, weiblich, 51, leidet an Blasenschwäche. Symptome: Kann den Urin nicht halten und starke Dringlichkeit zum Urinieren, Wasserlassen kurz und kraftlos. Bei Husten Tröpfchenverlust.

Nach Einnahme der Kräuter mit Rezept 7 über 3 Tage, spürt starke Verbesserung. Eine Therapie von einem Monat, kann Urin normal regulieren, über eine Therapiedauer von 3 Monaten symptomlos: Kann Urin normal regulieren und uriniert kräftig, fühlt sich körperlich gekräftigt und besitzt mehr Energie für den Alltag.

## Beschreibung

### Beschreibung der Ausführungsarten

Die Erfindung betrifft mehrere Kräutermischungen basierend auf TCM (Traditioneller Chinesischer Medizin), zur Verstärkung der Nieren- und Blasenfunktion gegen Blasenschwäche, sowie zur Leistungssteigerung. Die Erfindung unterstützt den Schliess- und Ringmuskel zu stärken für die verbesserte Kontrolle beim Harngang und Blutverdünnend wirkt und Kreislauffördernd.

In der TCM-Theorie sind Qi und Blut die beiden wichtigsten Substanzen im menschlichen Körper. Die Funktion von Qi besteht darin, die Bewegung des Blutes in den Blutgefäßen zu fördern. Blut transportiert Nährstoffe, nährt Organe und Gliedmaßen und produziert Qi, das die Durchblutung fördert. Dies ist das nährende Qi des menschlichen Körpers; das Qi treibt das Blut dazu, über die Oberfläche des menschlichen Körpers zu fließen, nährt die Haut und bildet eine Schutzbarriere, und dies ist das schützende Qi des menschlichen Körpers. Häufiges Wasserlassen und Harndrang aufgrund einer schwachen Blase bedeuten, dass Qi und Blut die Blase nicht ernähren können und das schützende Qi der Blase unzureichend ist, was zu einer Funktionsstörung der Blase und einer unzureichenden Zurückhaltung des Urins führt, was zu häufigem Wasserlassen und Harndrang führt. In der traditionellen chinesischen Medizin haben Alpiniae oxyphyllae fructus, Corni Fructus, Cornus officinalis Sieb. et Zucc., Cuscutae semen, Juglans regia, Morindae officinalis radix, Psoralea corylifolia Linn., Psoraleae fructus. Rosa davurica Pall. Fructus, Rosa laevigata Michx., Rosae laevigatae Fructus usw. die Funktion, die Niere zu stärken und das Wasserlassen zu reduzieren. Die vorliegende Erfindung kombiniert nierenverfestigende und den Harndrang reduzierende Arzneimittel mit der Förderung des Qi und der Beseitigung von Blutstauungen, um die Funktionen der Nieren und der Blase zu stärken und so die Vorstellung von Blasen- und Nierenfunktionsstörungen zu verbessern.

Die Erfindung umfasst jeweils 5-6 Zutaten. Diese kann zu Granulat verarbeitet werden und oral eingenommen.

Die Kräutermischung kann auch in Wasser gekocht als Tee verzehrt werden.

Die Zutaten werden zusammengemischt und zu Granulat verarbeitet als Tee, Kapsel- oder Tablettenform oral eingenommen. Die Substanzen der Zutaten können mit traditioneller chinesischer Medizin Technik mit Alkohol zu Konzentrat extrahiert werden.

Folgend sind die Wirkungen der Kräuter der traditionellen chinesischen Medizin beschrieben.

Folgende Kräuter wirken Nieren-, und Blasenstärkend:
- Alpiniae oxyphyllae fructus
- Corni Fructus
- Cornus officinalis Sieb. et Zucc.
- Cuscutae semen
- Juglans regia
- Morindae officinalis radix
- Psoralea corylifolia Linn.
- Psoraleae fructus
- Rosa davurica Pall. Fructus
- Rosa laevigata Michx.
- Rosae laevigatae Fructus
   Die Hauptwirkung der Kräuter ist die Nieren-, und Blasenfunktion zu stärken damit der Schliessmuskel gestärkt wird.

Folgende Kräuter wirken Blutverdünnend:
- Crocus sativus L
- Mori folium
- Paeonia lactiflora Pall.
- Persicae semen
- Rosae rugosae flos
- Salviae miltiorrhizae radix et rhizome
   Die Hauptwirkung der Kräuter ist das Blut zu verdünnen, für den erleichterten Bluttranport in den Gefässen.

Folgende Kräuter wirken Kreislauffördernd:
- Angelicae sinensis radix
- Astragali radix
- Chuanxiong rhizome
- Cinnamomi ramuli
- Citri reticulatae pericarpium viride
- Citrus reticulata Blanco
- Crataegus pinnatifida Bunge
- Piperis Longi Fructus
- Pricklyash Peel
   Die Hauptwirkung der Kräuter fördert das Qi, in der Sprache der Schulmedizin wird der Kreislauf gestärkt.

Eine Zusammensetzung mit Kräutern der traditionellen chinesischen Medizin gegen Nieren- und Blasenschwäche ist:
1. Astragali radix 7-12g, Paeonia lactiflora Pall. 7-15g, Crataegus pinnatifida Bunge 7-12g, Psoralea corylifolia Linn.5-12 g, Rosa laevigata Michx. 5-12g, Cornus officinalis Sieb. et Zucc. 5-12g
   Sechs weitere Zusammensetzungen mit Kräutern der traditionellen chinesischen Medizin gegen Nieren- und Blasenschwäche sind:
2. Piperis Longi Fructus 7-12g, Persicae semen 7-15g, Astragali radix, 5-9g, Psoraleae fructus 5-9g, Corni Fructus 7-12g
3. Chuanxiong rhizoma 7-12g, Paeonia lactiflora Pall. 7-15g, Angelicae sinensis radix 5-9g, Cuscutae semen7-12g, Corni Fructus 5-9g
4. Cinnamomi ramuli 7-12g, Salviae miltiorrhizae radix et rhizoma 7-15g, Angelicae sinensis radix 5-9g, Cuscutae semen 7-12g, Morindae officinalis radix 5-9g
5. Citri reticulatae pericarpium viride 7-12g, Crocus sativus L 3-7g, Angelicae sinensis radix 5-9g, Rosae laevigatae Fructus 7-12g, Corni Fructus 5-9g
6. Citrus reticulata Blanco 7-12g, Rosae rugosae flos 7-12g, Angelicae sinensis radix 5-9g, Alpiniae oxyphyllae fructus 7-12g, Juglans regia 5-9g
7. Pricklyash Peel 7-12g, Mori folium 7-15g, Angelicae sinensis radix 5-9g, Rosa davurica Pall. Fructus 7-12g, Alpiniae oxyphyllae fructus 5-9g

## Patentansprüche

1. Zusammensetzung der Kräutschermischung mit tradionellen TCM Kräutern Astragali radix 7-12g, Paeonia lactiflora Pall. 7-15g, Crataegus pinnatifida Bunge 7-12g, Psoralea corylifolia Linn.5-12 g, Rosa laevigata Michx. 5-12g, Cornus officinalis Sieb. et Zucc. 5-12g mit dem Effekt der Stärkung des Schliess- und Ringmuskels

2. Zusammensetzung der Kräutschermischung mit tradionellen TCM Kräutern Piperis Longi Fructus 7-12g, Persicae semen 7-15g, Astragali radix, 5-9g, Psoraleae fructus 5-9g, Corni Fructus 7-12g mit dem Effekt der Stärkung des Schliess- und Ringmuskels

3. Zusammensetzung der Kräutschermischung mit tradionellen TCM Kräutern Chuanxiong rhizoma 7-12g, Paeonia lactiflora Pall. 7-15g, Angelicae sinensis radix 5-9g, Cuscutae semen 7-12g, Corni Fructus 5-9g mit dem Effekt der Stärkung des Schliess- und Ringmuskels

4. Zusammensetzung der Kräutschermischung mit tradionellen TCM Kräutern Cinnamomi ramuli 7-12g, Salviae miltiorrhizae radix et rhizoma 7-15g, Angelicae sinensis radix 5-9g, Cuscutae semen 7-12g, Morindae officinalis radix 5-9g mit dem Effekt der Stärkung des Schliess- und Ringmuskels

5. Zusammensetzung der Kräutschermischung mit tradionellen TCM Kräutern Citri reticulatae pericarpium viride 7-12g, Crocus sativus L 3-7g, Angelicae sinensis radix 5-9g, Rosae laevigatae Fructus 7-12g, Corni Fructus 5-9g mit dem Effekt der Stärkung des Schliess- und Ringmuskels

6. Zusammensetzung der Kräutschermischung mit tradionellen TCM Kräutern Citrus reticulata Blanco 7-12g, Rosae rugosae flos 7-12g, Angelicae sinensis radix 5-9g, Alpiniae oxyphyllae fructus 7-12g, Juglans regia 5-9g mit dem Effekt der Stärkung des Schliess- und Ringmuskels

7. Zusammensetzung der Kräutschermischung mit tradionellen TCM Kräutern Pricklyash Peel 7-12g, Mori folium 7-15g, Angelicae sinensis radix 5-9g, Rosa davurica Pall. Fructus 7-12g, Alpiniae oxyphyllae fructus 5-9g mit dem Effekt der Stärkung des Schliess- und Ringmuskels

8. Zusammensetzung gemäss Anspruch 1-7, sowie dessen Extrakt oder als Granulat

9. Zusammensetzung gemäss Anspruch 1-7, in herkömmlicher Verarbeitungsweise als Tablette oder Serum

10. die Ansprüche 2-8 mit dem Effekt der Reinigung und Pflege der Blutgefässe

11. die Ansprüche 2-8 mit dem Effekt der Physischen Leistungssteigerung

12. die Ansprüche 2-8 mit dem Effekt der Stärkung der Nieren- und Blasenfunktion
